(19) Europäisches Patentamt
European Patent Office
Office européen des brevets

(11) Veröffentlichungsnummer: **0 215 382 B1**

(12) **EUROPÄISCHE PATENTSCHRIFT**

(45) Veröffentlichungstag der Patentschrift: **01.08.90**

(21) Anmeldenummer: **86112217.4**

(22) Anmeldetag: **04.09.86**

(51) Int. Cl.⁵: **C07D 487/04**, A01N 43/90 // (C07D487/04, 249:00, 239:00),(C07D487/04, 239:00, 231:00)

(54) **7-Amino-azolo[1,5-a]pyrimidine und diese enthaltende Fungizide.**

(30) Priorität: **17.09.85 DE 3533050**

(43) Veröffentlichungstag der Anmeldung: **25.03.87 Patentblatt 87/13**

(45) Bekanntmachung des Hinweises auf die Patenterteilung: **01.08.90 Patentblatt 90/31**

(84) Benannte Vertragsstaaten: **AT BE CH DE FR GB IT LI NL SE**

(56) Entgegenhaltungen:
**EP-A- 0 071 792**
**EP-A- 0 141 317**
**DE-A- 1 620 694**
**DE-A- 1 695 525**
**DE-A- 2 257 547**

(73) Patentinhaber: **BASF Aktiengesellschaft, Carl-Bosch-Strasse 38, D-6700 Ludwigshafen(DE)**

(72) Erfinder: **Graf, Hermann, Dr., Ginsterstrasse 15, D-6704 Mutterstadt(DE)**
Erfinder: **Wahl, Peter, Dr., Valentinianstrasse 8, D-6802 Ladenburg(DE)**
Erfinder: **Rentzea, Costin, Dr., Richard-Kuhn-Strasse 1-3, D-6900 Heidelberg(DE)**
Erfinder: **Sauter, Hubert, Dr., Neckarpromenade 20, D-6800 Mannheim 1(DE)**
Erfinder: **Ammermann, Eberhard, Dr., Sachsenstrasse 3, D-6700 Ludwigshafen(DE)**
Erfinder: **Pommer, Ernst-Heinrich, Dr., Berliner Platz 7, D-6703 Limburgerhof(DE)**

EP 0 215 382 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß 7-Amino-azolo[1,5-a]pyrimidine, insbesondere das 7-Amino-6-phenyl-5-methyl-[1,2,4]-triazolo-[1,5-a]pyrimidin, pharmakologische Eigenschaften besitzen (US-PS 2 553 500).

Es ist ferner bekannt, daß 7-Amino-azolo[1,5-a]pyrimidine, insbesondere das 7-Amino-6-(4-tertiär-butyloxy-1-butyl)-5-methyl-2-methyl-pyrazolo-[1,5-a]-pyrimidin, als fungiziden Wirkstoff zu verwenden (EP 141 317). Seine fungizide Wirkung ist jedoch nicht ausreichend.

Es wurde nun gefunden, daß neue, spezielle 7-Amino-azolo[1,5-a]pyrimidine der Formel

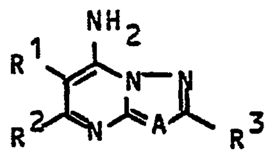

I,

worin
$R^1$ einen Phenoxy-$C_2$–$C_6$-alkoxy-$C_2$–$C_6$-alkyl-Rest oder einen Di-$C_1$–$C_{10}$-alkyl-amino-$C_2$–$C_6$-alkyl-Rest bedeutet, wobei der Phenylteil unsubstituiert oder ein- oder mehrfach mit geradkettigem oder verzweigtem $C_1$–$C_{10}$-Alkyl, Phenyl, $C_1$–$C_{10}$-Alkoxy, Phenoxy, Halogen, Phenyl-$C_1$–$C_4$-alkoxy, Di-$C_1$–$C_{10}$-alkyl-amino- oder $C_1$–$C_{10}$-Alkyl-phenylamino substituiert sein kann,
$R^2$ und $R^3$ für Wasserstoff oder $C_1$–$C_4$-Alkyl und
A für =N–, =CH–, =C($C_1$–$C_4$-Alkyl)–, =CBr– oder =CCl– steht,
die bekannten Verbindungen in ihrer fungiziden Wirkung, insbesondere bei Oomyceten, übertreffen.

$R^1$ bedeutet beispielsweise (Phenyl)-oxy-($C_2$- bis $C_6$)-alkoxy-($C_2$- bis $C_6$)-alkyl mit geradkettigen oder verzweigten Alkylenresten, bei denen Phenyl ein- oder mehrfach mit geradkettigem oder verzweigtem $C_1$- bis $C_{10}$-Alkyl, $C_1$- bis $C_{10}$-Alkoxy, Phenyl, Phenoxy, Fluor, Chlor, Brom, Phenyl-($C_1$- bis $C_4$)-alkyl, Phenyl-($C_1$–$C_4$)-alkoxy, Di-($C_1$–$C_{10}$-alkyl)-amino oder ($C_1$- bis $C_{10}$-Alkyl)-phenyl-amino substituiert sein kann;
$R^1$ kann weiter sein:
Di($C_1$- bis $C_{10}$-alkyl)-amino-($C_2$- bis $C_6$-)alkyl.

Für die Reste $R^2$ und $R^3$ stehen beispielsweise Wasserstoff oder $C_1$- bis $C_4$-Alkyl, wobei Methyl bevorzugt ist.

7-Amino-azolo[1,5-a]pyrimidine der Formel I erhält man beispielsweise, indem man einen entsprechend substituierten β-Ketoester der Formel II

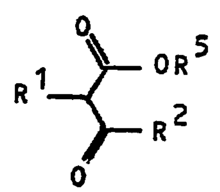

II,

($R^5$ steht für einen niederen Alkylrest) mit einem entsprechenden Amino-azol der Formel III

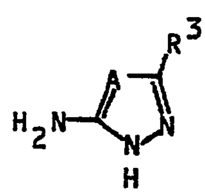

III,

zu Kondensationsprodukten der Formel V

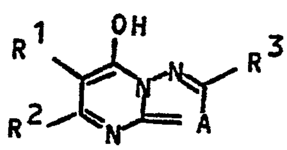

V,

umsetzt und diese an der Hydroxigruppe halogeniert und mit Ammoniak umsetzt (Verfahren A).

Die Darstellung der β-Ketoester (II) kann, wie in Organic Synthesis Coll. Vol. 1, S. 248, oder in DOS 3 227 388 beschrieben, durchgeführt werden. Ihre Umsetzung (Kondensation) mit den Aminoazolen (III) kann in Gegenwart oder Abwesenheit von Lösungsmitteln durchgeführt werden. Als Lösungsmittel kommen insbesondere Alkohole wie Ethanol, Propanole, Butanole, Glykole oder Glykolmonoether, Diethylenglykole oder deren Monoether, Amide wie Dimethylformamid, Diethylformamid, Dibutylformamid, N,N-Dimethylacetamid, niedere Alkansäuren wie Ameisensäure, Essigsäure, Propionsäure und Mischungen dieser Lösungsmittel mit Wasser in Frage. Die Umsetzungstemperatur liegt i.a. zwischen 50 und 300°C, vorzugsweise bei 50 bis 150°C, wenn in Lösung gearbeitet wird.

Die Kondensationsprodukte fallen meist in reiner Form an und werden nach dem Waschen (z.B. mit dem gleichen Lösungsmittel oder Wasser) und anschließendem Trocknen mit z.B. Phosphorhalogeniden, vorzugsweise bei 50 bis 150°C in überschüssigem Phosphoroxitrichlorid, bei Rückflußtemperatur halogeniert. Hierbei kann eine Base, beispielsweise N,N-Dimethyl-anilin, in stöchiometrischer Menge oder im Überschuß zugesetzt werden. Nach dem Verdampfen des überschüssigen Phosphoroxitrichlorids wird mit Eiswasser, gegebenenfalls unter Zusatz eines mit Wasser nicht mischbaren Lösungsmittels, behandelt und gegebenenfalls die Base durch Extraktion mit Salzsäure entfernt.

Das schließlich gewonnene Chlorierungsprodukt ist meist sehr rein und wird daher am besten unmittelbar mit Ammoniak zu den neuen 7-Amino-azolo[1,5-a]pyrimidinen umgesetzt. Dies geschieht vorzugsweise mit 1- bis 10-molarem Überschuß an Ammoniak unter Druck (bis zu 100 bar) oberhalb von etwa 100°C und ggf. in einem Lösungsmittel.

Die neuen 7-Amino-azolo[1,5-a]pyrimidine stellen sich i.a. als kristalline Verbindungen dar, die unmittelbar in gut reiner Form anfallen.

Man kann die 7-Amino-azolo[1,5-a]pyrimidine (I) auch herstellen, indem man entsprechend substituierte α-Acyl-nitrile der Formel

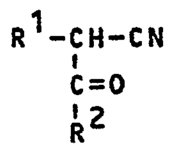

IV,

mit Aminoazolen der Formel (III) umsetzt (Verfahren B), wobei wiederum entweder ohne oder mit einem Lösungsmittel gearbeitet wird. Die Lösungsmittel und die Verarbeitungsbedingungen entsprechen weitgehend den für das Verfahren A empfehlenswerten. Bei dem Verfahren B erhält man die neuen 7-Amino-azolo[1,5-a]pyrimidine unmittelbar; sie werden, gegebenenfalls nach Verdampfen des Lösungsmittels oder Verdünnen mit Wasser, als kristalline, meist sehr reine Verbindungen isoliert. Bei Verwendung von niederen Alkansäuren (Fettsäuren) als Lösungsmittel ist es zweckmäßig, gegebenenfalls nach teilweisem Verdampfen des Überschusses, die Reste der Säure zu neutralisieren.

Die für die Herstellung der 7-Amino-azolo[1,5-a]pyrimidine benötigten substituierten α-Acyl-nitrile (VI) sind teilweise bekannt; im Einzelfalle unbekannte solche Nitrile können mit bekannten Methoden aus Nitrilen mit α-ständigem Wasserstoff und Carbonsäureestern mit starken Basen, z.B. Alkalihydriden, Alkaliamiden oder Metallalkylen hergestellt werden (J. Amer. Chem. Soc. 73, (1951), S. 3766).

Herstellbeispiel

Verfahren B

7-Amino-5-methyl-6-{3-[2-(2,4,6-trichlorphenoxy)-1-ethoxy]-1-propyl}-[1,2,4]triazolo[1,5-a]pyrimidin (Beispiel Nr. 8 der Tabelle)

a) 2-Acetyl-5-[2-(2,4,6-trichlorphenoxy)-1-ethoxy]-valeriansäurenitril

245 g (760 mmol) 5-[2-(2,4,6-trichlorphenoxy)-1-ethoxy]-valeriansäurenitril werden in 1 l trockenem Tetrahydrofuran gelöst und unter Schutzgas auf –68°C abgekühlt. Binnen 3 Stunden tropft man 572 ml 1,5-molare n-Butyllithium-Lösung in n-Hexan zu (entspricht 858 mmol n-Butyllithium) und rührt weitere 3 Stunden bei –60°C. Anschließend werden langsam 74,0 ml (66,7 g; 758 mmol) trockenes Ethylacetat, gelöst in 200 ml trockenem Tetrahydrofuran, zugegeben. Man beläßt wiederum 3 Stunden bei –60°C und läßt über Nacht auf Zimmertemperatur kommen. Durch vorsichtiges Zusetzen von Wasser wird überschüssiges Butyllithium vernichtet und durch Zugabe von 2 N-Salzsäure ein pH-Wert von vier eingestellt. Danach trennt man die organische Phase ab, wäscht mit Wasser, trocknet und engt ein. Als Rückstand verbleiben 267 g gelbes Öl (Rohausbeute 73%), das für die Umsetzung b) ohne weiteres eingesetzt werden kann.

b) Wirkstoff, entsprechend Beispiel 8 der Tabelle

Die Gesamtmenge (732 mmol) des, wie vorstehend beschrieben, hergestellten α-Acetyl-nitrils wird mit 61,5 g (731 mmol) 3-Amino-1H-1,2,4-triazol 24 Stunden lang in 1,0 l siedender Propionsäure gehalten. Danach läßt man abkühlen, filtriert und engt ein. Der Rückstand wird in Methylenchlorid aufgenommen,

mehrmals mit Wasser gewaschen, bis die wäßrige Phase neutral reagiert, getrocknet und eingedampft. Es resultieren 166 g (53%, bezogen auf das Nitril) kristallinen Materials (Schmp. 193–194°C).

Herstellbeispiel

Verfahren B

7-Amino-5-methyl-6-{2-[N-(3,5,5-trimethyl-1-hexyl)-N-methyl-amino]-1-ethyl}-[1,2,4]triazolo[1,5-a]pyrimidin (Beispiel Nr. 125)

a) 2-Acetyl-4-[N-(3,5,5-trimethyl-1-hexyl)-N-methyl-amino]-buttersäurenitril

31,3 g (139,5 mmol) 4-[N-(3,5,5-trimethyl-1-hexyl)-N-methyl-amino]-buttersäurenitril werden, wie vorstehend beschrieben, in 300 ml trockenem Tetrahydrofuran zuerst mit 103 ml 1,5 M n-Butyllithium-Lösung (154 mmol) und dann mit 13,7 ml (12,4 g; 141 mmol) trockenem Ethylacetat in 50 ml Tetrahydrofuran bei –68°C zur Reaktion gebracht. Bei der Aufarbeitung wird mit 2 N-Salzsäure pH 6 eingestellt. Nach Abdampfen des Solvens verbleiben 33,0 g (Rohausbeute 88%) eines Öls, das ohne weiteres für das Folgeprodukt verwendet wird.

b) Wirkstoff entsprechend Beispiel 125

Die Gesamtmenge (124 mmol) des erhaltenen Nitrils setzt man mit 10,4 g (124 mmol) 3-Amino-1H-1,2,4-triazol in 300 ml siedender Propionsäure während 18 Stunden um. Nach Entfernen des Lösungsmittels wird mit n-Pentan angerieben und abgesaugt. Man nimmt in Methylenchlorid auf und filtriert unter Zusatz von 5 Vol.-Prozent Methanol über eine kurze Kieselgelsäule. Das Eluat wird mit wäßriger Natriumcarbonatlösung ausgeschüttelt, getrocknet und eingeengt. 13,0 g (32%, bezogen auf das Nitril) Feststoff mit Schmp. 109–110°C bleiben zurück.

Nach den angegebenen Verfahren (A oder B) wurden die in den nachstehenden Tabellen näher charakterisierten (Schmelzpunkt, Aggregatzustand etc.) Wirkstoffe hergestellt. Die nicht näher charakterisierten Verbindungen können unter entsprechender Abwandlung der Rohstoffe und Anpassung der Herstellvorschriften leicht erhalten werden; sie lassen aufgrund ihrer strukturellen Ähnlichkeit eine vergleichbare Wirkung erwarten.

Tabelle 1 a

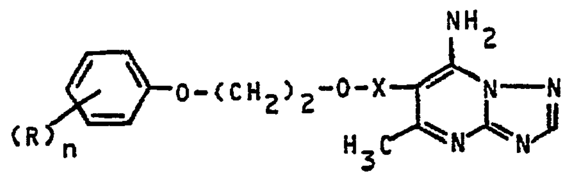

| Nr. | $(R)_n$ | -X- | Schmp. (°C) |
|---|---|---|---|
| 1 | H | $-(CH_2)_2-$ | |
| 2 | H | $-CH(CH_3)CH_2-$ | |
| 3 | H | $-(CH_2)_3-$ | 157-158 |
| 4 | H | $-(CH_2)_4-$ | |
| 5 | H | $-(CH_2)_5-$ | |
| 6 | 2,4,6-$Cl_3$ | $-(CH_2)_2-$ | |
| 7 | 2,4,6-$Cl_3$ | $-CH(CH_3)CH_2-$ | |
| 8 | 2,4,6-$Cl_3$ | $-(CH_2)_3-$ | |
| 9 | 2,4,6-$Cl_3$ | $-(CH_2)_4-$ | |
| 10 | 2,4,6-$Cl_3$ | $-(CH_2)_5-$ | |
| 11 | 2-Cl | $-(CH_2)_2-$ | |
| 12 | 2-Cl | $-CH(CH_3)CH_2-$ | |
| 13 | 2-Cl | $-(CH_2)_3-$ | |
| 14 | 2-Cl | $-(CH_2)_4-$ | |
| 15 | 2-Cl | $-(CH_2)_5-$ | |
| 16 | 4-Cl | $-(CH_2)_2-$ | |
| 17 | 4-Cl | $-CH(CH_3)CH_2-$ | |
| 18 | 4-Cl | $-(CH_2)_3-$ | |
| 19 | 4-Cl | $-(CH_2)_4-$ | |
| 20 | 4-Cl | $-(CH_2)_5-$ | |
| 21 | 3-Cl | $-(CH_2)_2-$ | |
| 22 | 3-Cl | $-CH(CH_3)CH_2-$ | |
| 23 | 3-Cl | $-(CH_2)_3-$ | 135-137 |
| 24 | 3-Cl | $-(CH_2)_4-$ | |
| 25 | 3-Cl | $-(CH_2)_5-$ | |
| 26 | 2-Br | $-(CH_2)_2-$ | |
| 27 | 2-Br | $-CH(CH_3)CH_2-$ | |
| 28 | 2-Br | $-(CH_2)_3-$ | 161-163 |
| 29 | 2-Br | $-(CH_2)_4-$ | |
| 30 | 2-Br | $-(CH_2)_5-$ | |
| 31 | 4-Br | $-(CH_2)_2-$ | |
| 32 | 4-Br | $-CH(CH_3)CH_2-$ | |
| 33 | 4-Br | $-(CH_2)_3-$ | |

| Nr. | $(R)_n$ | -X- | Schmp. (°C) |
|---|---|---|---|
| 34 | 4-Br | $-(CH_2)_4-$ | |
| 35 | 4-Br | $-(CH_2)_5-$ | |
| 36 | $2-CH_3$ | $-(CH_2)_2-$ | |
| 37 | $2-CH_3$ | $-CH(CH_3)CH_2-$ | |
| 38 | $2-CH_3$ | $-(CH_2)_3-$ | 164-166 |
| 39 | $2-CH_3$ | $-(CH_2)_4-$ | |
| 40 | $2-CH_3$ | $-(CH_2)_5-$ | 220 (Zers.) |
| 41 | $3-CH_3$ | $-(CH_2)_2-$ | |
| 42 | $3-CH_3$ | $-CH(CH_3)CH_2-$ | |
| 43 | $3-CH_3$ | $-(CH_2)_3-$ | 147-149 |
| 44 | $3-CH_3$ | $-(CH_2)_4-$ | |
| 45 | $3-CH_3$ | $-(CH_2)_5-$ | |
| 46 | $4-CH_3$ | $-(CH_2)_2-$ | |
| 47 | $4-CH_3$ | $-CH(CH_3)CH_2-$ | |
| 48 | $4-CH_3$ | $-(CH_2)_3-$ | 155-158 |
| 49 | $4-CH_3$ | $-(CH_2)_4-$ | |
| 50 | $4-CH_3$ | $-(CH_2)_5-$ | |
| 51 | $2,4,6-(CH_3)_3$ | $-(CH_2)_2-$ | |
| 52 | $2,4,6-(CH_3)_3$ | $-CH(CH_3)CH_2-$ | |
| 53 | $2,4,6-(CH_3)_3$ | $-(CH_2)_3-$ | 190-191 |
| 54 | $2,4,6-(CH_3)_3$ | $-(CH_2)_4-$ | |
| 55 | $2,4,6-(CH_3)_3$ | $-(CH_2)_5-$ | 157-160 |
| 56 | tert.-$C_4H_9-CH_2-C(CH_3)_2$ | $-(CH_2)_2-$ | |
| 57 | tert.-$C_4H_9-CH_2-C(CH_3)_2$ | $-CH(CH_3)CH_2-$ | |
| 58 | tert.-$C_4H_9-CH_2-C(CH_3)_2$ | $-(CH_2)_3-$ | |
| 59 | tert.-$C_4H_9-CH_2-C(CH_3)_2$ | $-(CH_2)_4-$ | |
| 60 | tert.-$C_4H_9-CH_2-C(CH_3)_2$ | $-(CH_2)_5-$ | 149-151 |
| 61 | $4-Cl-2-CH_3$ | $-(CH_2)_3-$ | 144-145 |
| 62 | $2-(i-C_3H_7)$ | $-(CH_2)_3-$ | |
| 63 | $2-(sec-C_4H_9)$ | $-(CH_2)_3-$ | 118-120 |
| 64 | $2-(sec-C_4H_9)$ | $-(CH_2)_5-$ | 154-156 |
| 65 | $4-C_6H_5$ | $-(CH_2)_3-$ | 176-179 |
| 66 | $4-C_6H_5$ | $-(CH_2)_5-$ | 172-174 |
| 67 | $4-H_5C_2O$ | $-(CH_2)_2-$ | 162-163 |
| 68 | $4-H_5C_2O$ | $-CH(CH_3)CH_2-$ | 158-160 |
| 69 | $4-H_5C_2O$ | $-(CH_2)_3-$ | |
| 70 | $4-H_5C_2O$ | $-(CH_2)_4-$ | |
| 71 | $4-H_5C_2O$ | $-(CH_2)_5-$ | |
| 72 | $4-H_5C_6O$ | $-(CH_2)_2-$ | |
| 73 | $4-H_5C_6O$ | $-CH(CH_3)CH_2-$ | |

| Nr. | $(R)_n$ | -X- | Schmp. (°C) |
|---|---|---|---|
| 74 | $4-H_5C_6O$ | $-(CH_2)_3-$ | 156-158 |
| 75 | $4-H_5C_6O$ | $-(CH_2)_4-$ | |
| 76 | $4-H_5C_6O$ | $-(CH_2)_5-$ | |
| 77 | $2-(n-C_4H_9)O$ | $-(CH_2)_3-$ | 133-135 |
| 78 | $2-(n-C_4H_9)O$ | $-(CH_2)_4-$ | |
| 79 | $2-(n-C_4H_9)O$ | $-(CH_2)_5-$ | |
| 80 | $3-(n-C_4H_9)O$ | $-(CH_2)_3-$ | |
| 81 | $3-(n-C_4H_9)O$ | $-(CH_2)_4-$ | |
| 82 | $3-(n-C_4H_9)O$ | $-(CH_2)_5-$ | |
| 83 | $4-(n-C_4H_9)O$ | $-(CH_2)_3-$ | |
| 84 | $4-(n-C_4H_9)O$ | $-(CH_2)_4-$ | |
| 85 | $4-(n-C_4H_9)O$ | $-(CH_2)_5-$ | |
| 86 | $2-(H_5C_6-CH_2)O$ | $-(CH_2)_3-$ | |
| 87 | $2-(H_5C_6-CH_2)O$ | $-(CH_2)_5-$ | |
| 88 | $3-(H_5C_6-CH_2)O$ | $-(CH_2)_3-$ | |
| 89 | $3-(H_5C_6-CH_2)O$ | $-(CH_2)_5-$ | |
| 90 | $4-(H_5C_6-CH_2)O$ | $-(CH_2)_3-$ | |
| 91 | $4-(H_5C_6-CH_2)O$ | $-(CH_2)_5-$ | |
| 92 | $3-(H_5C_2)N$ | $-(CH_2)_3-$ | |
| 93 | $3-(H_5C_2)N$ | $-(CH_2)_5-$ | |

<u>Tabelle 1 b</u>

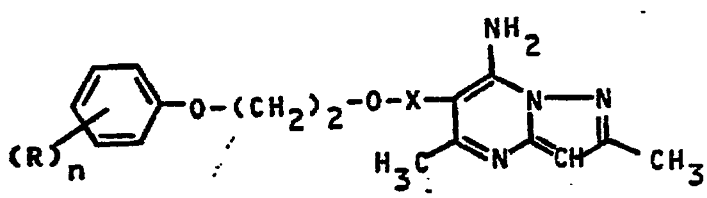

| Nr. | $(R)_n$ | -X- | Schmp. (°C) |
|---|---|---|---|
| 94 | $t-C_4H_9-CH_2-C(CH_3)_2-$ | $-(CH_2)_3-$ | 60 |
| 95 | $t-C_4H_9-CH_2-C(CH_3)_2-$ | $-(CH_2)_5-$ | (Öl) |

Tabelle 3

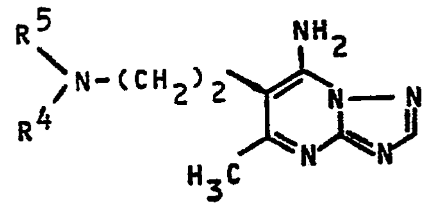

| Nr. | $R^5$ | $R^4$ | Schmp. (°C) |
|---|---|---|---|
| 124 | $n-C_6H_{13}-$ | $n-C_6H_{13}-$ | 139-140 |
| 125 | 3,5,5-Trimethylhexyl- | $CH_3-$ | 109-110 |

Tabelle 4

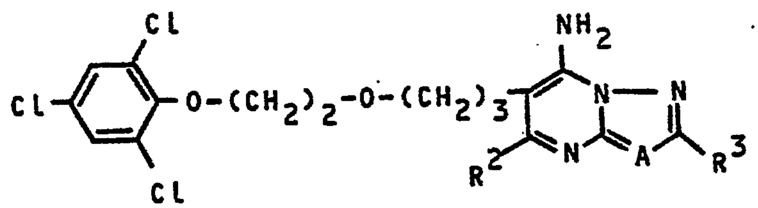

| Nr. | $R^2$ | $R^3$ | A | Schmp. (°C) |
|---|---|---|---|---|
| 126 | H | H | N | |
| 127 | $CH_3$ | $CH_3$ | N | |
| 128 | $CH_3$ | $CH_3$ | CH | |
| 129 | $CH_3$ | $CH_3$ | C-Br | |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben sowie von Erysiphe graminis an Getreide, Uncinula necator an Reben, Podosphaera leucotricha an Äpfeln, Sphaerotheca fuliginea an Rosen, Erysiphe cichoracearum an Gurken.

Die Wirkstoffe besitzen eine hohe Pflanzenverträglichkeit. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Die Bekämpfung der Pilze erfolgt in der Weise, daß die Pilze oder die vor Pilzbefall zu schützenden Materialien, Pflanzen, der Boden oder die Saatgüter mit einer fungizid wirksamen Menge der neuen Wirkstoffe behandelt werden.

Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 bis 5 kg Wirkstoff je ha.

Die Wirkstoffe können auch zusammen mit anderen Wirkstoffen, z.B. Herbiziden, Insektiziden, Wachstumsregulatoren und anderen Fungiziden, oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Die neuen Wirkstoffe werden beispielsweise in Form von direkt versprühbaren Lösungen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxymethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Ricinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung des Beispiels 3 mit 100 Gewichtsteilen N-Methylpyrrolidon und erhält eine Lösung, die zur Anwendung in Form kleinster Tropfen geeignet ist.

II. 20 Gewichtsteile der Verbindung des Beispiels 8 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der nach Beispiel 23 erhältlichen Verbindung werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der nach Beispiel 38 erhältlichen Verbindung werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

V. 20 Gewichtsteile der nach Beispiel 43 erhältlichen Verbindung werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der nach Beispiel 94 erhältlichen Verbindung werden mit 95 Gewichtsteilen feinteiligem Kaolin innig vermischt. Man erhält auf diese Weise ein Stäubemittel, das 5 Gew.% des Wirkstoffs enthält.

VIII. 40 Gewichtsteile der nach Beispiel 124 erhältlichen Verbindung werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der nach Beispiel 23 erhältlichen Verbindung werden mit 2 Teilen Calciumsalz der Dodecylbenzolsulfonsäure, 8 Teilen Fettalkoholpolyglykolether, 2 Teilen Natriumsalz eines Phenolsulfonsäureharnstoff-formaldehyd-Kondensats und 68 Teilen eines paraffinischen Mineralöls innig vermischt. Man erhält eine stabile ölige Dispersion.

Die folgenden Versuche belegen die biologische Wirkung der neuen Verbindungen. Zu Vergleichszwecken wurden die bekannten Wirkstoffe 7-Amino-6-phenyl-5-methyl-[1,2,4-triazol-[1,5-a]pyrimidin (A) (US 2 553 500) und 7-Amino-6-(4-tertiär-butyloxy-1-butyl)-5-methyl-2-methyl-pyrazolo-[1,5-a]pyrimidin (B) (EP 141 317) verwendet.

Versuch 1

Wirksamkeit gegen Plasmopara viticola

Blätter von Topfreben der Sorte "Müller-Thurgau" werden mit wäßriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise bei der Anwendung als 0,05%ige Wirkstoffbrühe die Wirkstoffe 3, 8, 23, 38, 43, 94 und 124 eine bessere fungizide Wirkung (beispielsweise 97%) zeigen als die bekannten Wirkstoffe (A) und (B) (beispielsweise 60%).

Versuch 2

Wirksamkeit gegen Phytophthora infestans an Tomaten

Blätter von Topfpflanzen der Sorte "Große Fleischtomate" werden mit wäßriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise bei der Anwendung als 0,025%ige Wirkstoffbrühe die Wirkstoffe 8, 63 und 124 eine bessere fungizide Wirkung zeigen (beispielsweise 97%) als der bekannte Wirkstoff B (0%).

**Patentansprüche**

1. 7-Amino-azolo[1,5-a]pyrimidine der Formel

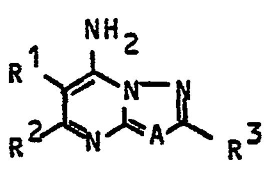

I,

worin

$R^1$ einen Phenoxy-$C_2$–$C_6$-alkoxy-$C_2$–$C_6$-alkyl-Rest oder einen Di-$C_1$–$C_{10}$-alkyl-amino-$C_2$–$C_6$-alkyl-Rest bedeutet, wobei der Phenylteil unsubstituiert oder ein- oder mehrfach mit geradkettigem oder ver-

zweigtem $C_1$–$C_{10}$-Alkyl, Phenyl, $C_1$–$C_{10}$-Alkoxy, Phenoxy, Halogen, Phenyl-$C_1$–$C_4$-alkyl, Phenyl-$C_1$–$C_4$-alkoxy, Di-$C_1$–$C_{10}$-alkyl-amino oder $C_1$–$C_{10}$-Alkyl-phenylamino substituiert sein kann,
$R^2$ und $R^3$ für Wasserstoff oder $C_1$–$C_4$-Alkyl und
A für =N–, =CH–, =C($C_1$–$C_4$-Alkyl)–, =CBR– oder =CCl– steht.

2. Fungizides Mittel, gekennzeichnet durch einen Gehalt an einem festen oder flüssigen Trägerstoff und einem 7-Amino-azolo[1,5-a]pyrimidin gemäß der Formel I gemäß Anspruch 1.

3. Verfahren zur Bekämpfung von Pilzen, dadurch gekennzeichnet, daß man die Pilze oder die vor Pilzbefall zu schützenden Materialien, Pflanzen, Boden oder Saatgüter behandelt mit einer fungizid wirksamen Menge eines 7-Amino-azolo[1,5-a]pyrimidins der Formel I gemäß Anspruch 1.

4. 7-Amino-5-methyl-6-{3-[2-(2,4,6-trichlorphenoxy)-ethoxy]-1-propyl)-[1,2,4]triazolo[1,5-a]-pyrimidin.

5. 7-Amino-5-methyl-6-[2-(N,N-dihexyl-amino]-1-ethyl]-[1,2,4]triazolo[1,5-a]pyrimidin.

6. 7-Amino-5-methyl-6-[3-(2-phenoxy-1-ethoxy)-1-propyl]-[1,2,4]triazolo[1,5-a]pyrimidin.

7. 7-Amino-5-methyl-6-[3-(2-(3′-chlorphenoxy-)-1-ethoxy)-1-propyl]-[1,2,4]triazolo[1,5-a]pyrimidin.

8. 7-Amino-5-methyl-6-[3-(2-(2′-methylphenoxy-)-1-ethoxy)-1-propyl]-[1,2,4]triazolo[1,5-a]pyrimidin.

9. 7-Amino-5-methyl-6-[3-(2-(3′-methylphenoxy-)-1-ethoxy)-1-propyl]-[1,2,4]triazolo[1,5-a]pyrimidin.

**Claims**

1. A 7-aminoazolo[1,5-a]pyrimidine of the formula

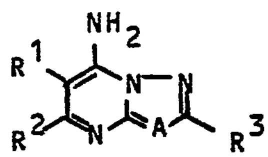

I,

where $R^1$ is di-$C_1$–$C_{10}$-alkylamino-$C_2$–$C_6$-alkyl phenoxy-$C_2$–$C_6$-alkoxy-$C_2$–$C_6$-alkyl or, in which the phenyl moiety is unsubstituted or monosubstituted or polysubstituted by straight-chain or branched $C_1$–$C_{10}$-alkyl, phenyl, $C_1$–$C_{10}$-alkoxy, phenoxy, halogen, phenyl-$C_1$–$C_4$-alkyl, phenyl-$C_1$–$C_4$-alkoxy, di-$C_1$–$C_{10}$-alkylamino or $C_1$–$C_{10}$-alkylphenylamino, $R^2$ and $R^3$ are each hydrogen or $C_1$–$C_4$-alkyl, and A is =N–, =CH–, =C($C_1$–$C_4$-alkyl)–, =CBR– or =CCl.

2. A fungicidal composition containing a solid or liquid carrier and a 7-aminoazolo[1,5-a]pyrimidine of the formula I as claimed in claim 1.

3. A method for controlling fungi, wherein the fungi or the materials, plants, soil or seed to be protected against fungal attack are treated with a fungicidally effective amount of a 7-aminoazolo[1,5-a]pyrimidine of the formula I as claimed in claim 1.

4. 7-amino-5-methyl-6-{3-[2-(2,4,6-trichlorophenoxy)-ethoxy]prop-1-yl}-1,2,4-triazolo[1,5-a]-pyrimidine.

5. 7-amino-5-methyl-6-[2-(N,N-dihexylamino)-eth-1-yl]-1,2,4-triazolo[1,5-a]pyrimidine.

6. 7-amino-5-methyl-6-[3-(2-phenoxy-1-ethoxy)-prop-1-yl]-1,2,4-triazolo[1,5-a]pyrimidine.

7. 7-amino-5-methyl-6-[3-(2-(3′-chlorophenoxy)-1-ethoxy)-prop-1-yl]-1,2,4-triazolo[1,5-a]pyrimidine.

8. 7-amino-5-methyl-6-[3-(2-(2′-methylphenoxy)-1-ethoxy)-prop-1-yl]-1,2,4-triazolo[1,5-a]pyrimidine.

9. 7-amino-5-methyl-6-[3-(2-(3′-methylphenoxy)-1-ethoxy)-prop-1-yl]-1,2,4-triazolo[1,5-a]pyrimidine.

**Revendications**

1. 7-amino-azolo[1,5-a]pyrimidine de formule

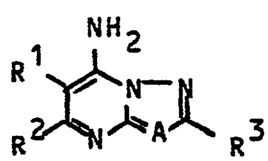

I,

dans laquelle
$R^1$ représente un reste phénoxy-alcoxy en $C_2$–$C_6$-alkyle en $C_2$–$C_5$ ou un reste Di-$C_1$–$C_{10}$-alkyl-amino-alkyle en $C_2$–$C_6$, la partie phényle étant non substituée ou pouvant être substituée une ou plusieurs fois par alkyle en $C_1$–$C_{10}$ halogène, phényl-alkyle en $C_1$–$C_4$, phényl-alcoxy en $C_1$–$C_4$, Di-alkyle en $C_1$–$C_{10}$ amino ou alkyle en $C_1$–$C_{10}$-phénylamino.

$R^2$ et $R^3$ sont mis pour hydrogène ou alkyle en $C_1$–$C_4$ et pour =N–, =CH–, =C (alkyle en $C_1$–$C_4$)–, =CBR– ou =CCl.

2. Agent fongicide caractérisé par une teneur en un support solide ou liquide et une 7-amino-azolo[1,5-a]pyrimidine de formule I selon la revendication 2.

3. Procédé pour lutter contre les champignons, caractérisé par le fait qu'on traite les champignons ou les matériaux, plantes, sols ou semences à protéger contre une attaque par champignons avec une quantité efficace du point de vue herbicide d'une 7-amino-azolo[1,5-a]pyrimidine de formule I selon la revendication 1.

4. 7-amino-5-méthyl-6-(3-[2-(2,4,6-trichlorophénoxy)-éthoxy]-1-propyl)-[1,2,4]triazolo[1,5-a]-pyrimidine.

5. 7-amino-5-méthyl-6-[2-(N,N-dihexyl-amino]-1-éthyl]-[1,2,4]triazolo[1,5-a]pyrimidine.

6. 7-amino-5-méthyl-6-[3-(2-phénoxy-1-éthoxy)-1-propyl]-[1,2,4]triazolo[1,5-a]pyrimidine.

7. 7-amino-5-méthyl-6-[3-(2-(3'-chlorophénoxy-)-1-éthoxy)-1-propyl]-[1,2,4]triazolo[1,5-a]pyrimidine.

8. 7-amino-5-méthyl-6-[3-(2-(2'-méthylphénoxy-)-1-éthoxy)-1-propyl]-[1,2,4]triazolo[1,5-a]pyrimidine.

9. 7-amino-5-méthyl-6-[3-(2-(3'-méthylphénoxy-)-1-éthoxy)-1-propyl]-[1,2,4]triazolo[1,5-a]pyrimidine.